# EUROPÄISCHE PATENTANMELDUNG

(11) **EP 1 004 338 A1**
(43) Veröffentlichungstag der Anmeldung: **31.05.2000**
(21) Anmeldenummer: 99120903.2
(22) Anmeldetag: 28.10.1999
(51) Int. Cl.: B01D 19/04, C12M 1/21, C02F 3/28

(54) **Verfahren zur Eindämmung der Schaumbildung bei Handhabung und Vergärung von vergärbarem organischem Material**

(30) Priorität: 04.11.1998 DE 19850822
(71) Anmelder: Wacker-Chemie GmbH, 81737 München (DE)
(72) Erfinder: Straubinger, Bernhard, Dr., 84539 Zangberg (DE); Menzel, Helmut, Dr., 84489 Burghausen (DE); Aboagye-Mathiesen, George, Dr., 8320 Marslet (DK); Juel, Holger, 2300 Kopenhagen S (DK)
(74) Vertreter: Fritz, Helmut, Dr.

(57) **Zusammenfassung**

Gegenstand der Erfindung ist ein Verfahren zur Eindämmung der Schaumbildung bei Handhabung und anaerober Vergärung von zu energieliefernden oxidierbaren Gasen vergärbarem organischem Material, bei welchem dem vergärbaren organischen Material Silicon-Antischaummittel zugesetzt wird.

## Beschreibung

Die Erfindung betrifft ein Verfahren zur Eindämmung der Schaumbildung bei Handhabung und Vergärung von vergärbarem organischem Material.

Aus anaerob vergärbarem organischem Material, insbesondere aus Abfällen, wie Gülle, Mist, Abfallfette und -Öle, organische Kantinen- und Industrieabfälle, Schlachthofabfälle und Kompost wird in Bioreaktoren verschiedenster Bauart mikrobiell Biogas hergestellt, welches vorwiegend aus Methan mit geringen Anteilen an Wasserstoff besteht. Das erhaltene Methan wird sodann in Generatoren zur Erzeugung von elektrischer Energie und Wärme verbrannt.

Bei Handhabung, wie Transport, Umfüllen und Vergärung kann es zu einer erheblichen Schaumbildung kommen. Die Schaumbildung stellt in den Biogasanlagen ein Problem dar. Der Gärungsprozess kann bei Schaumbildung nicht sicher gesteuert werden. Schaum kann aus den Behältern austreten. Dies führt zu erheblichen technischen Problemen, da Schaum, Flüssigkeit und zu vergärendes Material in die Gastanks gelangen oder sogar in den nachgeschalteten Generator eindringen kann. Dies kann erhebliche Schäden verursachen. Bei Überschäumen muß gegebenenfalls der Bioreaktor entleert und die Anlage gereinigt werden. Standzeiten sind die Folge. Ferner werden Nährstoffe in den Schaum befördert; dort sind diese für die Mikroorganismen weniger gut zugänglich, wodurch eine Beeinträchtigung der Gärung verursacht wird.

Meist wurde bisher versucht, den Schaum zu beherschen, indem weniger Flüssigkeit oder vergärbares organisches Material in die Bioreaktoren eingefüllt wurde. Weiterhin wurde der Schaum auf mechanische Weise eingedämmt. Es wurde versucht, Schaumkontrolle durch Zugabe des geeigneten Substrates, das wenig schäumt, zu erreichen. Auch wurden Bioreaktoren bei niedrigerer Temperatur betrieben. Alle diese Maßnahmen führen zu verminderter Produktivität.

Es bestand die Aufgabe, die Schaumbildung bei Handhabung und Vergärung von vergärbarem organischem Material einzudämmen.

Gegenstand der Erfindung ist ein Verfahren zur Eindämmung der Schaumbildung bei Handhabung und anaerober Vergärung von zu energieliefernden oxidierbaren Gasen vergärbarem organischem Material, bei welchem dem vergärbaren organischen Material Silicon-Antischaummittel zugesetzt wird.

Mit Hilfe der Silicon-Antischaummittel kann die Füllhöhe der Gärtanks der Bioreaktoren optimal genutzt werden. Der Einsatz von Silicon-Antischaummitteln führt zu einem stablieren Gärungsprozess. Durch optimale Schaumkontrolle kann der Gärprozess über einen weiten Temperaturbereich beherrscht werden. Neben den Vergärungen im sog. mesophilen Bereich bei , 20 - 45°C sind auch thermophile Kulturen und Bedingungen möglich, beispielsweise bei 45 - 80°C. Da man den Prozess zur Vergärung von vergärbarem organischem Material auch bei höherer Temperatur fahren kann, steigert man somit die Produktivität der Biogasanlage erheblich.

Ein weiterer Vorteil der Silicon-Antischaummittel ist deren Stabilität gegenüber den die Vergärung betreibenden Mikroben. Die Silicon-Antischaummittel werden von den Mikroben nicht metabolisiert, es findet also kein Abbau während des Gärprozesses statt. Die Silicon-Antischaummittel widerstehen den aggressiven Bedingungen und können über längere Zeit ihre Ewirkung entfalten.

Die Silicon-Antischaummittel zeigten keinerlei negativen Einfluß auf die Mikroben. Durch die biochemische Inertheit der Silicon-ASM ist ferner ausgeschlossen, daß irgendwelche Silicon-Antischaummittel den Fermentationsprozess in irgendeiner Weise stören könnten.

Das Silicon-Antischaummittel enthalt als wesentlichen Bestandteil, vorzugsweise mindestens 50 Gew.-%, insbesondere mindestens 70 Gew.-% Organopolysiloxan (A).

Vorzugsweise ist das Organopolysiloxan (A) aus Einheiten der allgemeinen Formeln (I) bis (VII)

R₃SiO_{1/2} (I),

R₂SiO (II) (II),

RSiO_{3/2} (III),

SiO_{4/2} (IV),

R₂ (R'O) SiO_{1/2} (V),

R(R'O) SiO (VI),

R' ORSiO_{3/2} (VII),

aufgebaut, worin
**R** einwertige, gegebenenfalls mit Halogenatomen, Cyano-, Amino-, Alkylamino-, quarternären Ammonium-, Mercapto-, Epoxy-, Anhydrido-, Carboxylato-, Sulfonato-, Sulfato-, Phosphonato-, Isocyanato- oder Polyoxyalkylengruppen substituierte Kohlenwasserstoffreste mit 1 bis 18 Kohlenstoffatomen und
**R'** einwertige, gegebenenfalls mit Halogenatomen, Cyano-, Amino-, Alkylamino-, quarternären Ammonium-, Mercapto-, Epoxy-, Anhydrido-, Carboxylato-, Sulfonato-, Sulfato-, Phosphonato-, Isocyanato- oder Polyoxyalkylengruppen substituierte Kohlenwasserstoffreste mit 1 bis 30 Kohlenstoffatomen und Wasserstoffatome bedeuten.

Beispiele für Kohlenwasserstoffreste **R** und **R'** sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Alkenylreste, wie der Vinyl-, Allyl- und der 5-Hexen-1-ylrest; Cycloalkylreste, wie Cyclopentyl-, Cyclohexyl-, Cycloheptylreste und Methylcyclohexylreste; Arylreste, wie der Phenyl-, Naphthyl- und Anthryl- und Phenanthrylrest; Alkarylreste, wie o-, m-, p-Tolylreste, Xylylreste und Ethylphenylreste; Aralkylreste, wie der Benzylrest, der alpha- und der β-Phenylethylrest.

Beispiele für substituierte Reste **R** und **R'** sind Cyanalkylreste, wie der β-Cyanethylrest, und mit Fluor, Chlor oder Bromatomen halogenierte Kohlenwasserstoffreste, beispielsweise Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m- und p-Chlorphenylrest.

Vorzugsweise sind mindestens 90 Mol-% der Reste **R** Methyl-, Ethyl- oder Phenylreste, insbesondere Methylreste.

Beispiele für die durch Polyoxyalkylengruppen substituierten Reste **R** und **R'** sind die Reste der allgemeinen Formel (VIII)

-R¹-[O(CR₂²)_{c}]_{d}OR² (VIII),

in der
**R**^{**1**} einen zweiwertigen C₁- bis C₆-Alkylenrest,
**R**^{**2**} Wasserstoffatome oder einwertige C₁- bis C₆-Kohlenwasserstoffreste,
**c** die Werte 0, 1, 2, 3, 4 oder 5, vorzugsweise 2 oder 3 und
**d** ganzzahlige Werte von 1 bis 100, vorzugsweise 1 bis 10 bedeuten.

Beispiele für die zweiwertigen Reste **R**^{**1**} sind gesättigte gerad- oder verzweigtkettige oder cyclische Alkylenreste wie der Methylen- und Ethylenrest sowie Propylen-, Butylen-, Pentylen-, Hexylen-, 2-Methylpropylen-, Cyclohexylenreste, oder ungesättigte Alkylenreste wie der Propenylen- und Hexenylenrest.

Beispiele für die einwertigen Reste **R**^{**2**} sind bei den vorstehenden Beispielen für **R** und **R'** aufgeführt.

Weitere Beispiele für die durch Polyoxyalkylengruppen substituierten Reste **R** und **R'** sind die Reste der allgemeinen Formel (IX) 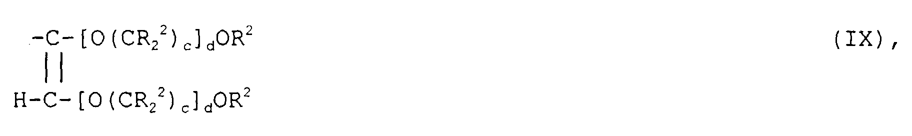 worin **R**^{**2**}**, c** und **d** die vorstehend für die allgemeine Formel (VIII) angegebenen Bedeutungen aufweisen.

Bevorzugte mit Alkylaminoresten substituierte Reste **R** und **R'** sind die Reste der allgemeinen Formel (X)

R⁵₂NR⁶- (X),

worin
**R**^{**5**} gleich oder verschieden sein kann und Wasserstoff oder einwertigen, gegebenenfalls halogensubstituierten C₁-C₁₀-Alkylrest oder C₁-C₁₀-Aminoalkylrest und
**R**^{**6**} einen zweiwertigen C₁-C₁₅-Kohlenwasserstoffrest bedeuten.

Beispiele für Rest **R**^{**5**} sind die für Rest **R** gegebenen Beispiele für C₁-C₁₀-Aminoalkylreste sowie C₁-C₁₀-Aminoalkylreste, wobei der Aminoethylrest besonders bevorzugt ist.

Vorzugsweise ist an jedes Stickstoffatom in den Resten der allgemeinen Formel (X) mindestens ein Wasserstoffatom gebunden.

Bevorzugt handelt es sich bei Rest **R**^{**6**} um zweiwertige Kohlenwasserstoffreste mit 1 bis 10 Kohlenstoffatomen, besonders bevorzugt 1 bis 4 Kohlenstoffatomen, insbesondere um den n-Propylenrest.

Beispiele für Rest **R**^{**6**} sind der Methylen-, Ethylen-, Propylen-, Butylen-, Cyclohexylen-, Octadecylen-, Phenylen- und Butenylenrest.

Bevorzugte Beispiele für Reste der allgemeinen Formel (X)sind
H₂N(CH₂)₃-,
H₂N(CH₂)₂NH(CH₂)₂-,
H₂N(CH₂)₂NH(CH₂)₃-,
H₂N(CH₂)₂-,
H₃CNH(CH₂)₃-,
C₂H₅NH(CH₂)₃-,
H₃CNH(CH₂)₂-,
C₂H₅NH(CH₂)₂-,
H₂N(CH₂)₄-,
H₂N(CH₂)5-,
H(NHCH₂CH₂)₃-,
C₄H₉NH(CH₂)₂NH(CH₂)₂-,
cyclo-C₆H₁₁NH(CH₂)₃-,
cyclo-C₆H₁₁NH(CH₂)₂-,
(CH₃)₂N(CH₂)₃-,
(CH₃)₂N(CH₂)₂-,
(C₂H₅)₂N(CH₂)₃- und
(C₂H₅)₂N(CH₂)₂-.

Beispiele für die quarternären Ammoniumreste sind die mit C₁-C₁₀-Aminoalkylresten quarternisierten Reste der allgemeinen Formel (X).

Vorzugsweise weisen höchstens 20 Mol-% der Einheiten des Organopolysiloxans (A) die allgemeinen Formeln (V) bis (VII) auf.

Vorzugsweise enthält das Organopolysiloxan (A) mindestens 50 Gew.-%, insbesondere mindestens 80 Gew.-% Organopolysiloxane (A1), welche zu mindestens 90 Mol-%, insbesondere 95 Mol-% aus Einheiten der allgemeinen Formel (II) bestehen. Dabei weisen vorzugsweise mindestens 50 Mol-% der restlichen Einheiten die allgemeinen Formeln (I) und (V) auf. Weiterhin ist bevorzugt, daß Organopolysiloxan (A1) eine durchschnittliche Viskosität von 50 bis 500 000 mPa.s, insbesondere von 350 bis 60 000 mPa.s bei 25°C aufweist.

Vorzugsweise enthält das Organopolysiloxan (A) mindestens 0,5 Gew.-%, insbesondere mindestens 2 Gew.-% und vorzugsweise höchstens 40 Gew.-% Organopolysiloxanharze (A2), welche zu mindestens 90 Mol-%, insbesondere 95 Mol-% aus Einheiten der allgemeinen Formeln (I), (IV) und (V) bestehen. Beispielsweise können die Organopolysiloxanharze (A2) bei Raumtemperatur fest sein und 0,25 bis 1,25 Einheiten der allgemeinen Formel (I) pro Einheit der allgemeinen Formel (IV) aufweisen. Diese bevorzugten Organopolysiloxanharze (A2) können, bedingt durch ihre Herstellung, bis zu insgesamt 5 Gew.-% Si-gebundene Alkoxyreste oder Hydroxylgruppen enthalten. Die Organopolysiloxanharze (A2) sind in der Regel mit Polydimethylsiloxanen nicht vollständig mischbar.

Das Silicon-Antischaummittel kann weiterhin noch Füllstoffe (B) enthalten. Beispiele für Füllstoffe (B) sind hydrophile und hydrophobe Oxide von Silicium, Magnesium oder Zink, wobei diese Oxide vorzugsweise jeweils eine Oberfläche von mindestens 50 m²/g aufweisen, Salze von Elementen der II. oder III. Gruppe des Periodensystems nach Mendeleieff mit einer Ordnungszahl von 12 bis 30 mit 12 bis 22 Kohlenstoffatome je Molekül aufweisenden, aliphatischen einbasigen Carbonsäuren oder Hydroxycarbonsäuren, wie Calciumstearat oder Calcium-12-hydroxystearat. Weitere Beispiele für Füllstoffe (B) sind Lithiumstearat, Magnesiumsilikat und Magnesiumaluminiumsilikat. Besonders bevorzugt ist pyrogen erzeugtes oder gefälltes insbesondere hydrophobiertes Siliciumdioxyd mit einer Oberfläche von mindestens 50 m²/g (hochdisperse Kieselsäure).

Die Silicon-Antischaummittel können eine Art von Füllstoft oder Gemische aus mindestens zwei verschiedenen Arten von Füllstoffen enthalten. Der Anteil von Füllstoff (B) beträgt vorzugsweise bis zu 20 Gewichtsteile, insbesondere 2 bis 8 Gewichtsteile pro 100 Gewichtsteile Organopolysiloxan (A).

Der Anteil von Füllstoff (B) beträgt vorzugsweise bis zu 20 Gewichtsteile, insbesondere 2 bis 8 Gewichtsteile pro 100 Gewichtsteile Organopolysiloxan (A).

Die Silicon-Antischaummittel können beispielsweise unverdünnt, in Form von Emulsionen oder in Form von Pulvern eingesetzt werden. Es können auch selbstemulgierende Silicon-Antischaummittel eingesetzt werden.

Silicon-Antischaumemulsionen haben gegenüber den wasserfreien Silicon-Antischaumkonzentraten folgende Vorteile: Das Silicon-Antischaummittel wird im vergärten organischen Material sehr schnell verteilt. Dadurch erreicht man eine sehr schnelle Schaumzerstörung. Dies ist insbesondere bei der "Stopping Method" (siehe Beispiele) von Bedeutung. Ferner ist eine sehr ökonomische Dosierung möglich. Bei den unverdünnten Silicon-Antischaummitteln kann es zu Überdosierungen kommen.

Wenn das Silicon-Antischaummittel in Form einer Emulsion eingesetzt wird, können beliebige für die Silicon-Emulsionen geeignete Tenside (C), verwendet werden. Es können auch Gemische mehrerer Tenside eingesetzt werden.

Der Anteil der Tenside (C) beträgt vorzugsweise höchstens 50, insbesondere höchstens 20 Gew.-Teile pro 100 Gewichtsteile Organopolysiloxan (A).

Die Silicon-Antischaummittel können mit oder ohne Wasserzusatz an Feststoffen ((D) adsorbiert bzw. in Feststoffen absorbiert werden, was ihre Formulierung als Pulver ermöglicht. Beispiele für solche Feststoffe (D) sind Kunststoffe, wie Polyvinylacetate, Polyvinylalkohole, Stärke, Polyacrylate, Zeolithe, anorganische Salze, wie Phosphate, beispielsweise Natriumtripolyphosphat und Trinatriumphosphat und Sulfate, wie Natriumsulfat.

Die Silicon-Antischaummittel lassen sich auch als solche oder vermischt mit geeigneten Zusatzstoffen (E) wie Pentandioldiisobutyrat oder in dispergierter Form mit Verdickungsmittel, beispielsweise Methylcellulosetypen, Carboxymethylcellulosen, Stärke, oder Polyvinylalkoholen und Polymerdispersionen, beispielsweise von Polyvinylacetat, sprühtrocknen.

Als Zusatzstoffe (E) kann das Silicon-Antischaummittel beispielsweise noch Fungizide, Bakterizide, Algicide, Biocide, Geruchsstoffe, Korrosionsinhibitoren, und, wenn auch nicht bevorzugt, organische Lösungsmittel enthalten.

Das Verfahren kann auf verschiedenste Art ausgeführt werden: Das Silicon-Antischaummittel kann präventiv eingesetzt werden, d.h. man gibt das Silicon-Antischaummittel schon von Anfang an - vor der Schaumbildung - dem vergärbaren organischen Materialsystem zu, indem man es vorlegt. In der Folge entfaltet das Silicon-Antischaummittel sodann seine entschäumende Wirkung. Hier wirkt das Silicon-Antischaummittel als Antischaummittel.
"Stopping Method": Nach erfolgter Schaumbildung wird der Schaum durch Silicon-Antischaummittel Zugabe zerstört, sodaß hier das Silicon-Antischaummittel auch als Entschäumer fungiert. Man kann das Silicon-Antischaummittel auch schon zu einem früheren Zeitpunkt dem vergärbaren organischen Material zumischen, welches z.B. zu den Anlagen transportiert wird. Dies hat den Vorteil, daß die Behälter (z.B. Sattelschlepper) effektiver mit vergärbaren organischen Material, wie Gülle gefüllt werden können, da während des Befüllens und des Transportes die Schaumbildung unterdrückt wird. Die Zugabe von Silicon-Antischaummittel ermöglicht somit eine bessere Volumenausnutzung von Behältern jeglicher Art für den Transport von vergärbarem organischen Material.

Durch Einsatz eines automatischen Dosiersystems bestehend aus Schaumsensor und Dosieranlage kann man in produkt- und zeitsparender Weise den Einsatz des Silicon-Antischaummittels weiter verbessern.

Die Antischaummittel-Wirkung des Silicon-Antischaummittels wird dann in die Vergärungsanlage weitergetragen.

Im Verfahren werden vorzugsweise 0,5 bis 500 g, insbesondere 2 bis 10 g Silicon-Antischaummittel auf 1m³ vergärbares organisches Material eingesetzt.

Das Verfahren kann in verschiedensten Anlagen zur Biogaserzeugung eingesetzt werden. Verschiedenste anaerobe Gärungsprozesse für vergärbares organisches Material fallen unter diese Anwendung. Besonders geeignet sind Biogasanlagen in beispielsweise Tank- oder Turmbauweise, Faul- und Klärschlammverarbeitung sowie Kompostieranlagen.

Das vergärbare organische Material kann beliebig sein und insbesondere aus Haushalten, Landwirtschaft, Handwerk und Industrie stammen. Beispielsweise kann das vergärbare organische Material Abfälle, wie Gülle, Mist, Abfallfette und - Öle, organische Kantinenabfälle, Abfälle aus der Getränke-, Lebensmittel und Tierfutter herstellenden und auch - verarbeitenden Industrie, wie Abfälle aus der zuckerverarbeitenden Industrie, z.B. Melasse-Abfälle, Abfälle aus der Stärkeproduktion, Brauereiabfälle, Abfälle aus Fermentationsbetrieben, Schlachtabfälle, Abfälle aus fischverarbeitenden Betrieben und Kompost umfassen.

In den nachstehend beschriebenen Beispielen beziehen sich alle Angaben von Teilen und Prozentsätzen, falls nicht anders angegeben, auf das Gewicht. Des weiteren beziehen sich alle Viskositätsangaben auf eine Temperatur von 25°C. Sofern nicht anders angegeben, wurden die nachstehenden Beispiele bei einem Druck der umgebenden Atmosphäre, also etwa 1000 hPa, und bei Raumtemperatur, also bei etwa 20°C, bzw. bei einer Temperatur, die sich beim Zusammengeben der Reaktanden bei Raumtemperatur ohne zusätzliche Heizung oder Kühlung einstellt, durchgeführt.

### Beispiele

### Eingesetzte Silicon-Antischaummittel Emulsionen

Emulsion 1 besteht aus:
   - 9,5% aus einem Organopolysiloxan der Komponenten Polydimethylsiloxan der Viskosität 350 mPa.s Harz, bestehend aus Me₃SiO_{1/2} und SiO_{4/2}-Einheiten α,w-alkoxyfunktionelles Siliconöl
   - 0,5 % hochdisperse Kieselsäure mit einer BET-Oberfläche > 50m²/g
   - 7% aus nichtionogen Tensiden folgender Zusammensetzung: 4% einer Mischung von Estern eines mehrwertigen Alkohols mit einer Fettsäure 3% ethoxylierte Fettalkohole
   - 83 % Wasser
Emulsion 2 besteht aus:
   a) 18 % aus Organopolysiloxan der Komponenten:
      * Polydimethylsiloxan mit Viskosität 1000 mPa.s
      * Harz, bestehend aus Einheiten Me₃SiO_{1/2} und SiO_{4/2} * α,ω-alkoxyfunktionelles Siliconöl
   b) Emulgatoren
      * 1 % nichtionischer Emulgator
      * 2 % anionischer Emulgator
      * 4 % Fettsäureester
   c) Kieselsäure
      * 2 % hochdisperse Kieselsäure, BET-Oberfläche > 50 m²/g
   d) 73 % Wasser
Emulsion 3 besteht aus:
   a) 90 % Organopolysiloxan der Komponenten:
      * Polydimethylsiloxan mit Viskosität 1000 mPa.s
      * Harz, bestehend aus den Einheiten Me₃SiO_{1/2} und SiO_{4/2}
      * α,ω-alkoxyfunktionelles Siliconäl
   b) Kieselsäure
      * 10 % hochdisperse Kieselsäure, BET-Oberfläche > 50 m²/g

### Beispiel 1 ("Stopping Method"und präventiv)

In einen Bioreaktor wurden als vergärbares organisches Material täglich 12 m³Schweinegülle, welche 300 bis 500 l Fischölabfälle enthielt, eingespeist. Bei der Zersetzung der Fischölabfälle entstanden Tenside, die sich an der Grenzfläche Biomasse/Biogas anreichern und starkes Schäumen verursachen.
Kontinuierlich wurden 0,5 bis 1 l Silicon-Antischaummittel Emulsion 1 in 150 m³ Biomasse pro Tag gegeben. Dadurch wurde im gesamten Testzeitraum die Schaumbildung wirksam unterdrückt. Zusätzlich wurden die entstanden Tenside, welche auch Zwischenprodukte für die anaeroben Bakterien waren, besser verteilt. Dadurch wurde die Biomasse besser verwertet als ohne die Zugabe von Silicon-Antischaummittel Emulsion 1. Die Produktivität in Bezug auf Biogas erhöhte sich um 4,3 bis 43 % im Vergleich zum Betrieb ohne Silicon-Antischaummittel 1.

### Beispiel 2("Stopping Method")

Vergärbare Haushaltsabfälle, die hohe Anteile an Tensiden aufwiesen und deshalb stärker schäumten als das vergärbare organische Material von Beispiel 1, wurden in einer Biogasanlage eingesetzt.
Zweimal pro Woche wurden 2 bis 3 Liter Silicon-Antischaummittel Emulsion zum gärenden Material in einen Reaktor* von 2.100 m³ zugegeben. Der Schaum wurde wirksam unterdrückt.
* Gesamtes Reaktorvolumen: 2.100 m³
Durchgesetzte Biomasse/Gülle pro Woche: 702 m³

### Beispiel 3 ("Stopping Method")

In einer Anlage, bestehend aus drei Biogasreaktoren mit je 200 m³ Volumen, war ein permantentes Schaumproblem gegeben. In jeden Reaktor wurden täglich 5 - 10 l Antischaummittel zugegeben:
- Reaktor 1:: Silicon-Antischaummittel Emulsion 1
- Reaktor 2:: Silicon-Antischaummittel Emulsion 2
- Reaktor 3:: Silicon-Antischaummittel Emulsion 3
Der Schaum wurde durch Antischaummittelzugabe jeweils für mehrere Stunden unterdrückt.

## Patentansprüche

1. Verfahren zur Eindämmung der Schaumbildung bei Handhabung und anaerober Vergärung von zu energieliefernden oxidierbaren Gasen vergärbarem organischem Material, bei welchem dem vergärbaren organischen Material Silicon-Aritischaummittel zugesetzt wird.

2. Verfahren nach Anspruch 1, bei dem das Silicon-Antischaummittel als wesentlichen Bestandteil Organopolysiloxan (A) enthält, welches aus Einheiten der allgemeinen Formeln (I) bis (VII)
R₃SiO_{1/2} (I),
R₂SiO (II),
RSiO_{3/2} (III),
SiO_{4/2} (IV),
R₂(R'O)SiO_{1/2} (V),
R(R'O)SiO (VI),
R'ORSiO_{3/2} (VII),
aufgebaut ist, worin
**R** einwertige, gegebenenfalls mit Halogenatomen, Cyano-, Amino-, Alkylamino-, quarternären Ammonium-, Mercapto-, Epoxy-, Anhydrido-, Carboxylato-, Sulfonato-, Sulfato-, Phosphonato-, Isocyanato- oder Polyoxyalkylengruppen substituierte Kohlenwasserstoffreste mit 1 bis 18 Kohlenstoffatomen und
**R'** einwertige, gegebenenfalls mit Halogenatomen, Cyano-, Amino-, Alkylamino-, quarternären Ammonium-, Mercapto-, Epoxy-, Anhydrido-, Carboxylato-, Sulfonato-, Sulfato-, Phosphonato-, Isocyanato- oder Polyoxyalkylengruppen substituierte Kohlenwasserstoffreste mit 1 bis 30 Kohlenstoffatomen und Wasserstoffatome bedeuten.

3. Verfahren nach Anspruch 1 oder 2, bei dem das Organopolysiloxan (A) mindestens 50 Gew Organopolysiloxane (A1) enthält, welche zu mindestens 90 Mol-% aus Einheiten der allgemeinen Formel (II) bestehen.
